# EUROPEAN PATENT SPECIFICATION

(11) **EP 1 830 917 B1**
(45) Date of publication and mention of the grant of the patent: **05.06.2013**
(21) Application number: 04800263.8
(22) Date of filing: 02.11.2004
(51) Int. Cl.: A61N 1/08, A61N 1/372, A61B 5/0452, A61B 5/029

(54) **DEVICE FOR EVALUATING POSITIONS OF AN IMPLANTABLE MEDICAL DEVICE**
VORRICHTUNG ZUR BEURTEILUNG DER POSITIONEN EINES IMPLANTIERBAREN MEDIZINPRODUKTS
DISPOSITIF POUR EVALUER LES POSITIONS D'UN DISPOSITIF MEDICAL IMPLANTABLE

(43) Date of publication of application: 12.09.2007
(73) Proprietor: St. Jude Medical AB, 175 84 Järfälla (SE)
(72) Inventor: LJUNGSTRÖM, Karin, S-165 70 Hässelby (SE); BJÖRLING, Anders, 169 37 Solna (SE); STRANDBERG, Hans, S-172 65 Sundbyberg (SE)
(74) Representative: Sjölander, Henrik
(86) International application number: PCT/SE2004/001600
(87) International publication number: WO 2006/049538

(56) References cited:
- US-A- 5 129 394
- US-A1- 2002 045 809
- US-A1- 2002 045 809
- US-A1- 2004 024 425
- US-A1- 2004 116 975
- US-B1- 6 496 715

## Description

### TECHNICAL FIELD

The present invention generally relates to implantable medical devices such as implantable cardiac pacemakers and in particular to a device and a method for obtaining information related to the heart pumping activity at different lead positions within a heart and for using the information to evaluate the different lead positions in order to identify the optimal position with respect to the heart pumping activity.

### BACKGROUND OF THE INVENTION

The technology of cardiac pacemakers has developed in sophistication and functionality over the years. In general, cardiac pacemakers are designed to control the heart by correcting or compensating for various heart abnormalities which can be encountered in human patients. For example, cardiac pacemakers may provide therapeutic stimulation to the heart by delivering therapeutic pulses such as pacing, cardioversion or defibrillation pulses.

Commonly, the pulses are delivered to the heart via electrodes disposed on implantable leads coupled to the pacemaker. The pacemaker performs various sensing and pulsing functions by receiving and delivering signals through the leads. The placements of the electrodes, i.e. the leads, with respect to one or more cardiac locations is such so that the desired electrical functions such as stimulation or heart signal sensing are ensured. For example, the leads may position the electrodes with respect to one or more cardiac locations so that the pacemaker can deliver pulses to the appropriate locations of the heart.

Leads may be placed in one or more of a variety of different cardiac locations. In particular, the placement of the leads may be dependent on the cardiac conditions of the patient and the therapy to be delivered.

A proper placement of the leads, i.e. the electrodes, is essential because both the desired electrical functions, i.e. stimulation or heart signal sensing, and the desired heart muscle reaction (activity propagation) are dependent on the position of the lead. However, it is often difficult to determine whether a lead has been properly positioned and adequate tissue contact has been achieved. Today, pacemaker leads are normally checked during the implantation to ensure satisfactory electrical performance by use of a PSA (pacemaker system analyzer). A PSA is an external equipment connected to the implanted leads. In general three electrode and lead properties are checked by use of a PSA to be satisfactory:
1. Stimulation threshold. That the required electrical stimulation energy to activate the heart is low enough.
2. Heart signal. That the spontaneous heart signal picked up by the lead system is of enough amplitude.
3. Lead impedance. That the conductive path of the lead(s) together with body fluids and tissue is in physical good condition.

Furthermore, it is often difficult to get the lead to the proper or desired site, specially the left ventricular lead through the coronary sinus, partly due to technical difficulties and anatomic differences, but also due to the lack of a standard procedure for identifying the optimal or best stimulation spot for that specific patient. One method for left ventricular lead placement includes so called venogram, where a fluoro-visible dye is injected into the veins so that the veins are visible using a fluoroscopic device.

Thus, there is a need of an improved device and method that, in an efficient way, is capable of identifying the optimal position of the lead or the leads within a heart with respect to the heart pumping activity during an implantation procedure of an implantable medical device.

US 2002/0045809 discloses a system for mapping a heart comprising a mapping catheter having an ultrasonic position sensor and a reference catheter having an ultrasonic position sensor placeable in a fixed position relative to the heart. The position of the mapping catheter is determined relative to the reference catheter.

### BRIEF DESCRIPTION OF THE INVENTION

Thus, an object of the present invention is to provide a simplified and improved system for finding the optimal lead placement for one or more leads with respect to heart pumping activity during an implantation procedure.

This and other objects are achieved according to the present invention by providing a device having the features defined in the independent claim 1. Preferable embodiments of the invention are characterised by the dependent claims.

In the context of the present invention, the term "mechanical sensor" relates to sensors capable of providing one or more signals indicative of cardiac mechanical activity, such as acoustic sensors, accelerometer-based sensors, pressure sensors, etc..

According to a first aspect of the present invention, there is provided a device for evaluating positions of a medical lead during an implantation procedure, the medical lead comprising at least one electrode for stimulating and sensing, the medical lead comprising at least one mechanical sensor, the sensor being arranged to provide signals related to heart pumping activity. The device comprises measuring means arranged to record sensor signals at different lead positions; storage means arranged to store the sensor signals; and processing means arranged to determine the lead position resulting in the most favourable hemodynamics of heart based on a measure of the heart activity at each position.

Exemplary, however not forming part of the invention, there is provided a method for evaluating positions of a medical lead during an implantation procedure, wherein the medical lead comprises at least one electrode for stimulating and sensing, and at least one mechanical sensor, which sensor is arranged to provide signals related to heart pumping activity. The method comprises the steps of: recording sensor signals at different lead positions; storing the sensor signals; and determining the lead position resulting in the most favourable hemodynamics of heart based on a measure of the heart activity at each position.

Exemplary, however not forming part of the invention, there is provided a computer readable medium comprising instructions for bringing a programmable device to perform a method according to the second aspect of the invention.

Thus, the invention is based on the idea of utilizing signals characteristic of the heart pumping activity to evaluate different positions of a medical lead or medical leads during an implantation procedure in order to identity the optimal site or placement for the lead or the leads with respect to a desired heart muscle reaction. That is, physiologic parameters reflecting hemodynamic performance are derived and evaluated for each lead position in order to determine the optimal site.

This solution provides several advantages over the existing solutions. One advantage is that the optimal site for placing the lead (or the leads) can be accurately determined with respect to anatomic differences of a specific patient, the specific therapy to be delivered and the desired heart muscle reaction (activity propagation).

Another advantage is that the lead site optimization is incorporated into the implant procedure. This provides for significant time savings in comparison with prior art solutions, which, in turn, reduces the risk for infection because the time required for the implantation procedure is decreased.

In a preferred embodiment of the present invention, the lead or the leads incorporate one or more pressure sensors. The pressure sensors transduce hemodynamic pressure variations, for example, left ventricular pressure and/or right ventricular pressure, so as to provide one or more signals indicative of the heart pumping activity, which signals are used to determine, for example, a measure of the heart pumping activity such as pre-ejection time, left ventricular ejection time, or the ventricular contractility (peak left ventricular pressure change during systole or LV + dp/dt).

In accordance with another preferred embodiment of the present invention, the lead or the leads incorporate one or more accelerometer-based sensors, for example, cardiac wall motion sensors. The cardiac wall motion sensors transduce accelerations of cardiac tissue to which the leads are attached, so as to provide one or more signals indicative of cardiac mechanical activity, which can be used to derive physiologic parameters indicative of cardiac performance, including stroke volume, contractility, pre-ejection period, and ejection time, which can be used as a measure of heart pumping activity.

According to an embodiment of the present invention, the lead position resulting in the shortest pre-ejection time period (PEP) is determined to be the lead position resulting in the most favourable hemodynamics of the heart, wherein PEP is determined as the period from the onset of a QRS or an emitted ventricular stimulation until the opening of the aortic valve. The opening of the aortic valve is determined to the moment when a maximum of the time derivative of the ventricular pressure (i.e. dp/dt) occurs after the onset of a QRS complex or an emitted ventricular stimulation. In one embodiment, a pressure sensor is placed tranvenously through the coronary sinus and located in the coronary vein in order to sense the left ventricular pressure.

According to another embodiment of the present invention, the lead position resulting in the lowest value of the quotient between PEP and left ventricular ejection time (LVET) is determined to be the lead position resulting in the most favourable hemodynamics of the heart. LVET is affected by the contractility of the myocardium and by outflow obstructions at the left ventricle. At a degraded contractility with a low stroke volume LVET will decrease, whilst it will be lengthen at outflow obstructions, such as aortic stenosis, and at a large central stroke volume. PEP tends to increase at, inter alia, cardiac insufficiency. As with PEP, LVET must be corrected with respect to the heart rate. The PEP/LVET quotient reflects the function of the left ventricle in a more efficient way than the individual components and is not dependent on the heart rate. Hence, the quotient PEP/LVET provides an efficient and reliable measure of the heart pumping activity.

According to other embodiments of the present invention, the co-ordination of the contraction of the left ventricle and the right ventricle, respectively, or, in other words, the timing difference or delay between the contraction of the left ventricle and the right ventricle, respectively, is recorded at each lead position and the lead position resulting in the shortest delay between the contraction of the left ventricle and the contraction of the right ventricle is determined to be the lead position resulting in the most favourable hemodynamics of the heart. Preferably, the pressure in the coronary vein, which pressure is proportional to the left ventricular pressure, and the right ventricular pressure are sensed. Alternatively, the proportionality between the left ventricular pressure and the right ventricular pressure is utilized when optimizing the lead position or the lead positions.

According to still another embodiment of the present invention, the ventricular contractility (peak left ventricular pressure change during systole or LV + dp/dt) is recorded at each lead position and the lead position resulting in the highest LV + dp/dt is determined to be the lead position resulting in the most favourable hemodynamics of the heart. Because the left ventricle systolic performance directly determines the ability of the heart to pump blood through the systemic circulation, it has been found that an efficient way to assess the heart activity is to examine how well the left ventricle contracts in order to determine the effectiveness of the left ventricle as a pump. Therefore, by using the left ventricle contraction effectiveness or "contractility" as the measure of the heart pumping activity, the lead position can be optimized in an efficient and reliable way.

According to a further embodiment of the present invention, the cardiac output is determined at each lead position and the lead position resulting in the highest cardiac output, which is the volume of blood in litres ejected by the heart per minute, is determined as the lead position resulting in the most favourable hemodynamics of the heart.

Preferably, the AV interval between stimulation of the atrium and the ventricle and/or the VV interval between stimulation of the right and left ventricles are optimised before the measurement related to the determination of the performance of the actual lead position is performed.

As realized by the person skilled in the art, the disclosed methods are suitable to realize or implement as a computer program or a computer readable medium, preferably within the contents of a device in accordance with the first aspect of the present invention and in particular within the processing means of such a device.

Further objects and advantages of the present invention will be discussed below by means of exemplifying embodiments.

### BRIEF DESCRIPTION OF THE DRAWINGS

In the following detailed description, reference will be made to the accompanying drawings, of which:
Fig. 1 is a schematic diagram of a device for evaluating positions of a medical lead during an implantation according to one embodiment of the present invention;
Fig. 2 is schematic diagram of a Graphically User Interface which can be presented on a display of the device shown in Fig. 1 according to one embodiment of the present invention;
Fig. 3 is a flow chart describing the principle steps of the process of evaluating different position of a lead of an implantable medical device to find an optimal lead site in accordance with the present invention.

### DETAILED DESCRIPTION OF THE INVENTION

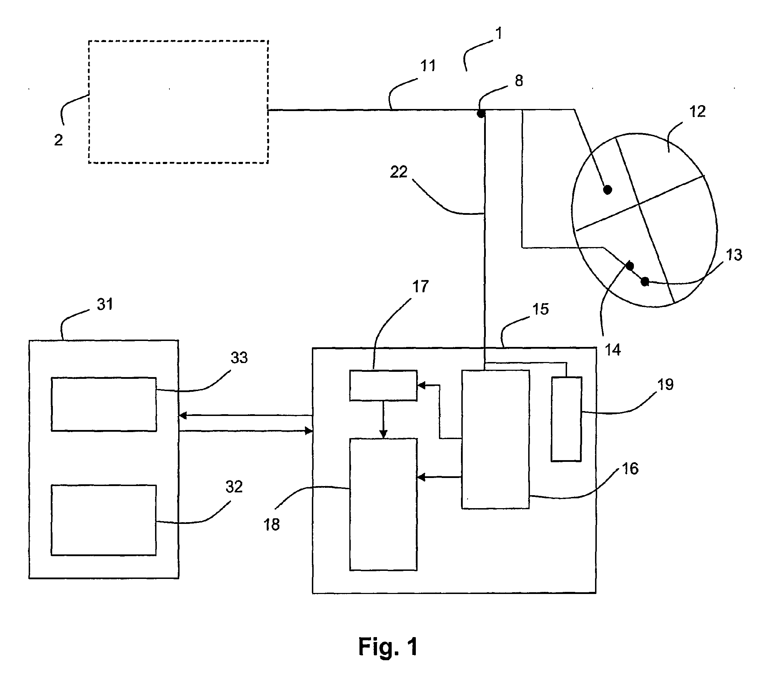

Fig. 1 is a diagram illustrating an implantable medical device (IMD) and a device for evaluating positions of a medical lead during an implantation procedure in order to find an optimal lead placement according to an embodiment of the present invention. IMD 1 comprises a lead 11, which may be unipolar or bipolar, and may be adapted to operate in cooperation with a wide variety of implantable medical devices.

In Fig. 1, one position of lead 11 in the heart 12 of a patient is shown. The lead 11 may include any of the passive or active fixation mechanisms known in the art for fixation of the lead to the cardiac tissue when final position has been found. As an example, lead distal tip (not shown) may include a tined tip or a fixation helix. The lead 11 comprises one or more electrodes 13, such as a tip electrode or a ring electrode. The electrode 13 senses electrical signals associated with depolarization and repolarization of the heart 12, and thereby can IEGM signals be obtained. In addition, the electrode 13 may also transmit pacing pulses for causing depolarization of cardiac tissue adjacent to the electrode 13. Furthermore, the lead 11 also comprises sensing means 14 arranged to sense signals related to the pumping activity of the heart 12. The sensing means may include different types of mechanical sensors that can be used with the present invention will be discussed below.

The IMD 1 is connectable to an active implantable medical device 2, for example, a pacemaker and after completion of the evaluation procedure and after positioning of the at least one electrode 13 at the final position, the IMD 1 is connected to the active implantable medical device 2. The active implantable medical device 2 comprises a pacemaker circuit (not shown), a processor (not shown), coupled to the at least one electrode 13, and a memory circuit (not shown) coupled to the processor. The pacemaker circuit comprises, among other things, a pulse generator circuit, a timer and control circuit coupled to the pulse generator circuit and the processor, and a telemetry circuit. The telemetry circuit, which when implanted telemetrically communicates with an external programmer, is coupled within the pacemaker to the memory circuit and the timing and control circuit. The operation and function of the different circuits of the IMD 2 is well-known for the man skilled in the art for what reason they are not described in further detail in the context of this application.

The evaluation device 15 comprises a measuring means 16, storage means 17, processing means 18, and a pulse generator 19. The storage means 17 may include a random access memory (RAM) and/or a non-volatile memory such as read-only memory (ROM). As will be appreciated by one of ordinary skill in the art, storage means may include various types of physical devices for temporary and/or persistent storage of data which includes solid state, magnetic, optical and combination devices. For example, the storage means may be implemented using one or more physical devices such as DRAM, PROMS, EPROMS, EEPROMS, flash memory, and the like. Furthermore, the processing means 18 may be a microprocessor and may also include storage means, which may include a random access memory (RAM) and/or a non-volatile memory such as read-only memory (ROM). The pulse generator 19 is connected to the lead 11 and is controlled by the processing means 18 and arranged to generate stimulation pulses for deliverance to the cardiac tissue adjacent to the electrode 13 of the lead 11.

According to an embodiment, the evaluation device 15 is detachably connected to the lead 11 during the implantation procedure at a connecting terminal 8 of the lead 11. The connecting terminal 8 is preferably adapted to the guide wire used during the implantation to place the lead 11 at different positions within the heart 12. In the embodiment described above and shown in Fig. 1, the measuring means is connected to the lead 11 or to the leads via the connection terminal 8 and a connection lead 22.

In an alternative embodiment, the device 15 is provided with one or more leads 11. The lead 11 comprises one or more electrodes 13, such as a tip electrode or a ring electrode. The electrode 13 senses electrical signals associated with depolarization and repolarization of the heart 12. In addition, the electrode 13 may also transmit pacing pulses for causing depolarization of cardiac tissue adjacent to the electrode 13. Furthermore, the lead 11 also comprises sensing means 14 arranged to sense a signals related to the pumping activity of the heart 12. The sensing means may include different types of sensors that can be used with the present invention as will be discussed below.

The measuring means 16 is arranged to measure or record the sensor signals and signals characteristic of the heart pumping activity at the current lead position by means of the sensing means 14 and/or the electrode 13 when the evaluation device 15 is connected directly to the lead 11 or the leads at the connection terminal 8 (or terminals if the are more than one lead) via the connection lead 22. Furthermore, the sensor signals and/or signals characteristic of the heart activity are stored in the storage means 17. The processing means 18 is connected to the measurement means 16 and is arranged, inter alia, to determine a measure of the heart pumping activity resulting from a stimulation pulse delivered to the cardiac tissue as current lead position based on the signals measured or recorded by the measuring means 16. The procedure for determining the measure will be described in further detail below.

In addition, the evaluation device 15 comprises an operator unit 31 as can be seen in Fig.1. The operator unit 31 comprises a keyboard 32, which allows the operator to input, for example, control commands, and a display or screen 33 for presenting information related to the evaluation of the obtained sensor signals from the different lead positions in or at heart 12. In an another embodiment, the screen 33 is a touch sensitive screen and the user can perform operations such as the above mentioned by touching the screen 33. The touch sensitive screen may include a number of soft-keys in order to present different commands at different presented interfaces on the screen 33. Of course, the touch sensitive screen can be combined with a keyboard and positioning device. According to an embodiment, storage means may be arranged and/or in the operator unit 31. The embodiment described above and shown in fig. 1 can be integrated within a standard pacemaker system analyzer (PSA), which is connected to the lead or the leads of the medical device by adding a measuring means 16 and include adequate processing software.

Alternatively, the operator unit 31 may, as the man skilled within the art realizes, be a stand-alone unit which is connectable to the evaluation device 15, the implantable medical device or the PSA. During the implantation procedure, the physician can, for example, use the keyboard 32 of the operator unit 31 to activate the pulse generator 19 to deliver a stimulation pulse when the lead has been placed on a site which is to be evaluated. In case of a touch sensitive screen, the pulse generator can be activated using, for example, a soft-key displayed on the screen 33.

Of course, there are a plurality of other conceivable embodiments, and the above described embodiments should be considered only as exemplifications. For example, the operator unit 31 may be a personal computer or a laptop computer.

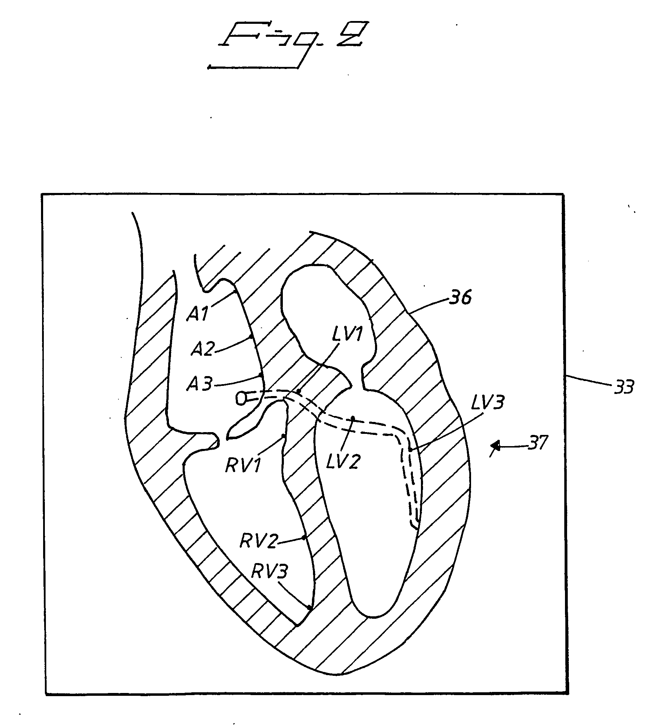

Turning now to Fig. 2, a Graphically User Interface which can be presented on the screen 33 is shown. The operator unit 31 may be arranged to present a Graphically User Interface on the screen 33 containing a diagrammatic picture 36 of the heart 12 of the patient in which the IMD 1 and the lead or the leads are about to implanted into. The user can perform operations on the picture of the heart 36 such as rotating the heart or enlarging or reducing parts thereof. These operations can, for example, be performed by means of the keyboard 32 or a positioning device such as a mouse. As mentioned above, the screen 33 may be a touch sensitive screen and the user can perform operations such as the above mentioned by touching the screen 33 and the touch sensitive screen may include a number of soft-keys in order to present different commands at different presented interfaces on the screen 33.

The physician or the attendant personnel can input the present position or positions for the lead or the leads, for example, in right atrium A1, A2, A3, in left ventricle LV1, LV2, LV3, and/or in right ventricle RV1, RV2, RV 3 on the screen 33 by entering the position data for the lead or the leads using the keyboard 32. Alternatively, the physician or the attendant personnel can by means of a cursor 37 controlled either by the keyboard 32 or a positioning device such as a mouse point out the present lead and electrode positions in right atrium A1, A2, A3, in left ventricle LV1, LV2, LV3, and/or in right ventricle RV1, RV2, RV 3 on the screen 33. Moreover, in case of a touch sensitive screen, the position or the positions can be input by touching the screen 33 at the proper position thereby indicating the present position or positions, for example, in right atrium A1, A2, A3, in left ventricle LV1, LV2, LV3, and in right ventricle RV1, RV2, RV 3. The position information or data can be obtained by means of a number of different methods, for example, X-ray, or ultrasound. Alternatively, the physician may gain knowledge of the actual position by personal feeling from his or hers fingers during the implantation procedure. Furthermore, position data or information of each lead positions is also stored in the storage means of operator unit 31 or the storage means 17 the evaluation device 15, which can be performed either automatically at inputting the position data or manually using, for example, the keyboard 32. As will be discussed in more detail below, a measure of score value of the heart pumping activity is calculated or determined for each lead position and the measure or value is stored together with the position data for the corresponding position. Subsequently, when a measure or score has been determined for all lead positions, the processing means determines the lead position resulting in the most favourable hemodynamics of the heart or, in other words, the most efficient heart activity, as will be discussed in greater detail below. The measure for each lead position together with the lead position resulting in the most favourable hemodynamics of the heart can be presented for the user, for example, the physician, visually on the screen 33 of the operator unit 31. Thereby, the user can easily identify the best or optimal position or site for the lead with respect to the heart pumping activity in a clear way. These measures can, for example, be presented on the screen 33 by means of a graphical method such as colour intensity or a bar diagram. For example, a high score or measure, i.e. a favourable hemodynamics of the heart, may be indicated with a dark colour and a lower score with a paler colour.

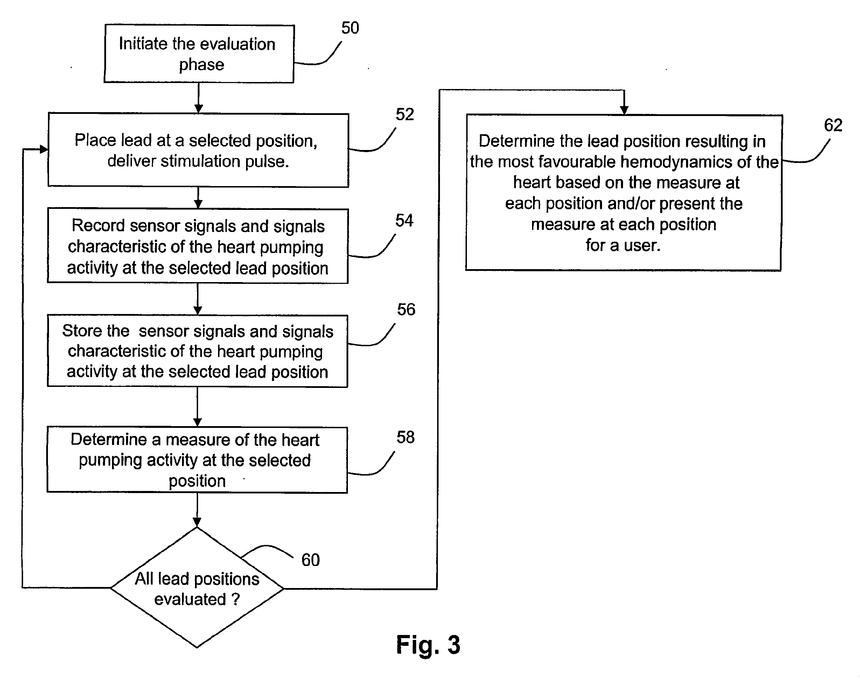

With reference now to Fig.3, a flow chart of the principles of the process of evaluating different position of one or more leads according to the present invention during implantation of the medical device will be described. First, at step 50, a test phase is activated by the user. This can be performed by activating an evaluation or optimization sequence or program stored in the storage means 17 of the evaluation device 15 or a storage means of the operator unit 31 by a start command input by means of the keyboard 32. For example, an activation of the evaluation sequence may result in that the Graphically User Interface, as described above, is presented on the screen 33 containing a diagrammatic picture 36 of a heart. As discussed above, the position or the placement of the lead is essential for the functions of the medical device as well as regards to obtaining the desired heart muscle reaction and finding or identifying the optimal lead position with respect to the heart activity is often difficult. Therefore, a number of different lead positions may have to be tested and evaluated during the implantation procedure of the medical device in order to find a placement of the lead that gives the optimal heart activity at stimulation. Moreover, the sensor or sensors are positioned at the appropriate positions with respect to the signals required to obtain the specific measure utilized to evaluate the lead positions.

Then, at step 52, the physician or an attendant personal places the lead 11 at a first position by means of the guide wire, a stimulation pulse is delivered at the selected position using the pulse generator 19. This can be performed manually by the physician or by an attendant personal by inputting a activation instruction using, for example, the keyboard 32 or the screen 33 to the processing means 18 instructing it to prompt the pulse generator 19 to deliver a pulse to the cardiac tissue of heart 12 at the position of the electrode 13. Alternatively, the pulse can be delivered on an automatic basis. For example, the pulse generator 19 can be prompted by the processing means 18 to deliver the pulse when a user by means of, for example, the keyboard 32 or the screen 33 states that the lead 11 has been placed on a position which shall be evaluated with respect to the heart pumping activity resulting from a stimulation pulse.

Thereafter, at step 54, sensor signals and signals characteristic of the heart activity are recorded. For example, it may be signals indicating the left ventricular and/or right ventricular pressure. That is, physiologic parameters reflecting hemodynamic performance are derived for each lead position. In addition, IEGM signals may also be recorded. As will be discussed in more detail below, there are a number of different parameters that can be used as hemodynamical indicators of the heart pumping activity including the pre-ejection period (PEP), the quotient between PEP and left ventricular ejection time (LVET), the coordination between the contraction of the left ventricle and the contraction of the right ventricle and/or the best proportion between the left ventricular pressure and the right ventricular pressure, the ventricular contractility (peak left ventricular pressure change during systole or LV + dp/dt), or the cardiac output. Subsequently, at step 56, the sensor signals and/or signals characteristic of the heart activity at the selected lead position is stored in the storage means of the evaluation device 15. As will be discussed below, the signal characteristic of the heart activity may depend on which parameter that is used in the evaluation procedure. Furthermore, position data or information of the actual lead position is also obtained and stored in the storage means. As discussed above, this position data may be obtained by means of a number of different methods, for example, X-ray, ultrasound, or by means of the physician by personal feeling from his or hers fingers during the implantation procedure. Preferably, the AV interval between stimulation of the atrium and the ventricle and/or the W interval between stimulation of the right and left ventricles are optimised before the measurement related to the determination of the performance of the actual lead position is performed.

At step 58, the processing means determines a measure or a score value of the heart activity for the actual lead position using the recorded signal data. The measure or score value may be presented for the user at the screen 33, for example, as a numerical value or as a graphical representation. At step 60, the user may select whether another lead position is to be evaluated or tested. If yes, the above mentioned steps 52-58 are repeated. If no, the processing means determines which lead position that results in the most favourable hemodynamics of the heart based on the determined or calculated measure for each lead position at step 62. As will be discussed below, a number of different parameters can be used for this determination. For example, the lead position resulting in the shortest pre-ejection time period (PEP) may be determined to be the optimal site with respect to the hemodynamics of the heart. The measure at each lead position together with the lead position resulting in the most favourable hemodynamics of the heart can be presented for the user visually at the screen 33 of the operator unit 31. Thereby, the user can easily identify the best or optimal position or site for the lead with respect to the desired heart activity in a clear way. These measures can, for example, be presented on the screen 33 by means of a graphical method such as colour intensity or a bar diagram.

In the following, different embodiments of the present invention employing different approaches to derive physiologic parameters reflecting hemodynamic performance for different lead positions in order to determine the optimal lead site will be described.

According to one embodiment of the present invention, the pre-ejection period at each lead position is measured and the lead position resulting in the shortest pre-ejection time period (PEP) is determined to be the lead position resulting in the most favourable hemodynamics of the heart. The processing means 18 is arranged to determine the PEP as the period from the onset of a QRS or an emitted ventricular stimulation until the opening of the aortic valve. Information regarding the onset of a QRS or an emitted ventricular stimulation is recorded by means of the measuring means 16 and transferred to the processing means 18. The sensing means 14 includes a sensor arranged to sense the opening of the aortic valve or the ejection of the left ventricle (LV). This may be achieved by measuring variations of the volume, detecting variations in the hemodynamic pressure of the left ventricular, or otherwise detecting the opening of the valve in a reliable manner. In this embodiment, the sensing means 14 includes a pressure sensor arranged to detect variations in the pressure within the left ventricular. The pressure sensor is placed tranvenously through the coronary sinus and located in the coronary vein. The pressure that is sensed in this location is proportional to the left ventricular pressure. The design and function of the pressure sensor is described in the U.S. Pat. No. 5,129,394. During the evaluation procedure, the measuring means 16 records the signals representative of the pressure variations at each lead position, the signals are stored in the storing means 17 and transferred to the processing means 18, which is arranged to determine the opening of the aortic valve to the moment when a maximum of the time derivative of the ventricular pressure (i.e. dp/dt) occurs after the onset of a QRS complex or an emitted ventricular stimulation. The processing means 18 determines the PEP for each lead position, which are used as a measure of the heart activity, where the information regarding the position of the lead is input into the evaluation device 15, for example, by the physician or by attendant personnel via the operator unit 31 as discussed above. As also mentioned above, the results can be presented for the physician and the attendant personnel during the implantation procedure on the screen 33. Then, the processing means 18 is arranged to determine the lead position resulting in the shortest PEP as the lead position resulting in the most favourable hemodynamics of the heart. As PEP tends to increase at, inter alia, cardiac insufficiency, therefore, a correction of the measured PEP with respect to the heart rate may have to be performed.

According to another embodiment of the present invention, the quotient between PEP and left ventricular ejection time (LVET) is determined and the lead position resulting in the lowest value of the quotient between PEP and left ventricular ejection time (LVET) to be the lead position resulting in the most favourable hemodynamics of the heart. The processing means 18 is arranged to determine the LVET as the period of time required for the systolic ejection of the left ventricle (LV) and the PEP as the period from the onset of a QRS or an emitted ventricular stimulation until the opening of the aortic valve. LVET depends on the heart rate and must therefore be corrected to it. Generally, LVET is specified as the percentage of the standard value of the heart rate. The standard value of LVET is about 92 - 108 %. LVET is affected by the contractility of the myocardium and by outflow obstructions at the left ventricle. At a degraded contractility with a low stroke volume LVET will decrease, while it will be lengthen at outflow obstructions, such as aortic stenosis, and at a large central stroke volume. PEP, as mentioned above, tends to increase at, inter alia, cardiac insufficiency. As with LVET, PEP must be corrected with respect to the heart rate. The PEP/LVET quotient reflects the function of the left ventricle in a more efficient way than the individual components and is not dependent on the heart rate. The standard value is about 0.30 - 0.40 for adults. At cardiac insufficiency, the PEP/LVET quotient can increase significantly and may reach values as high as 0.60. In this embodiment, the sensing means 14 includes a pressure sensor placed tranvenously through the coronary sinus and located in the coronary vein. The pressure that is sensed in this location is proportional to the left ventricular pressure. The design and function of the pressure sensor is described in the above mentioned U.S. Pat. No. 5,129,394. Furthermore, the measuring means 16 records the signals representative of the pressure variations, the signals are stored in the storing means 17 and transferred to the processing means 18, which is arranged to determine the opening of the valve to the moment when a maximum of the time derivative of the ventricular pressure occurs after the onset of a QRS complex or an emitted ventricular stimulation. Information regarding the onset of a QRS or an emitted ventricular stimulation is recorded by means of the measuring means 16 and transferred to the processing means 18. The sensing means 14 also includes a sensor for detecting the closing of the aortic valve. This may be achieved by measuring variations of the volume, detecting variations in the hemodynamic pressure of the left ventricular, or otherwise detecting the opening of the valve in a reliable manner. According to this embodiment, the pressure sensor of the sensing means 14, which detects variations in the pressure within the left ventricular, is also used to detect the closing of the aortic valve. The measuring means 16 records the signals representative of the pressure variations, the signals are stored in the storing means 17 and transferred to the processing means 18, which is arranged to determine the closing of the aortic valve to the moment when a maximum of the time derivative of the ventricular pressure (dp/dt) occurs. That is, at the maximum rate of rise of the ventricular pressure. Then, the processing means 18 determines the PEP and the LVET for each lead position in order obtain the PEP/LVET quotient for each position, which is used as a measure of the heart activity. The information regarding the position of the lead is input into the evaluation device 15, for example, by the physician or by attendant personnel via the operator unit 31 as discussed above. As also mentioned above, the results can be presented for the physician during the implantation procedure on the screen 33. Finally, the processing means 18 is arranged to determine the lead position resulting in the lowest value of the quotient between PEP and LVET to be the lead position resulting in the most favourable hemodynamics of the heart.

According to yet another embodiment of the present invention, the co-ordination of the contraction of the left ventricle and the right ventricle, respectively, or, in other words, the timing difference or delay between the contraction of the left ventricle and the right ventricle and/or the proportion between the left ventricular pressure and the right ventricular pressure is (are) measured at each lead position. The processing means 18 is arranged to determined the lead position resulting in the shortest delay between the contraction of the left ventricle and the contraction of the right ventricle and/or the proportion closest to a predetermined proportion, which predetermined proportion may be selected or defined by the user, between the left ventricular pressure and the right ventricular pressure to be the lead position resulting in the most favourable hemodynamics of the heart. It is known that a lack of synchronism between left and right ventricular side is inefficient and even destructive. In a first approximation synchronous timing is the goal. However, a short delay might be optimal, but that will be shown in clinical studies. Anyhow, the suggested embodiment facilitates processing of measured signals to set an optimal timing and/or lead position although a specific optimal value is not known.

In this embodiment, the sensing means 14 includes a pressure sensor placed transvenously through the coronary sinus and located in the coronary vein. The pressure that is sensed in this location is proportional to the left ventricular pressure. The design and function of the pressure sensor is described in the above mentioned U.S. Pat. No. 5,129,394. Moreover, the sensing means of a second lead includes a right ventricular pressure sensor located in the right ventricle arranged to sense variations of the right ventricular pressure. The measuring means 16 records the signals representative of the pressure variations of the left ventricle and the right ventricle, the signals are stored in the storing means 17 and transferred to the processing means 18. Then, the processing means 18 compares the pressure of the left and right ventricle, respectively, for each lead position in order to determine the co-ordination of the contraction of the left and right ventricle, respectively, and/or the proportionality between the left ventricular pressure and the right ventricular pressure. Accordingly, the delay or timing difference between the contraction of the left ventricle and right ventricle and/or the proportionality between the left ventricular pressure and the right ventricular pressure are determined for each lead position, which are used as a measure of the heart activity, wherein the information regarding the position of the lead is input into the evaluation device 15, for example, by the physician or by attendant personnel via the operator unit 31 as discussed above. As mentioned above, the results can be presented for the physician and the attendant personnel during the implantation procedure on the screen 33. The processing means 18 is arranged to determine the lead position resulting in the best co-ordination, i.e. the shortest delay between the contraction of the left ventricle and right ventricle, and/or the best proportionality between the left ventricular pressure and the right ventricular pressure as the lead position resulting in the most favourable hemodynamics of the heart.

According to still another embodiment of the present invention, the ventricular contractility (peak left ventricular pressure change during systole or LV + dp/dt) is measured at each lead position. The processing means 18 is arranged to determine the lead position resulting in the highest LV + dp/dt as the lead position resulting in the most favourable hemodynamics of the heart. The left ventricle systolic performance directly determines the ability of the of the heart to pump blood through the systemic circulation and one way to assess the heart activity is to examine how well the left ventricle contracts in order to determine the effectiveness of the left ventricle as a pump. One measure of left ventricle contraction effectiveness is called "contractility", which is the measure used in this embodiment. The left ventricle contractility is estimated by the processing means 18 using the peak positive rate of change of the left ventricular pressure during the systole, i.e. the maximum positive derivative of the left ventricular pressure. It should however be noted that the left ventricular dp/dt max is a widely accepted index of the contractile performance of the left ventricle. In this embodiment, the sensing means 14 includes a sensor arranged to detect variations in the pressure within the left ventricular. The pressure sensor is placed tranvenously through the coronary sinus and located in the coronary vein. The pressure that is sensed in this location is proportional to the left ventricular pressure. The design and function of the pressure sensor is described in the above mentioned U.S. Pat. No. 5,129,394. It should be noted that there are possible means for estimating the left ventricle systolic performance, for example, by measuring the stroke volume, which is the volume of blood pumped out of the left ventricle per systole. In order to achieve this a sensor for sensing the aortic pulse pressure can be used. During the evaluation procedure, the measuring means 16 records the signals representative of the pressure variations obtained from the pressure sensor at each lead position, the signals are stored in the storing means 17 and transferred to the processing means 18. The processing means 18 determines the LV + dp/dt at each lead position. The information regarding the positions of the lead is input into the evaluation device 15, for example, by the physician or by attendant personnel via the operator unit 31 as discussed above. As also mentioned above, the results can be presented for the physician during the implantation procedure on the screen 33. Then, when LV + dp/dt have been determined for all lead positions, the processing means 18 is arranged to determine the lead position resulting in the highest LV + dp/dt value to be the lead position resulting in the most favourable hemodynamics of the heart.

According to a further embodiment of the present invention, the cardiac output is determined at each lead position. The processing means 18 is arranged to determine the lead position resulting in the highest cardiac output, which is the volume of blood in litres ejected by the heart per minute, as the lead position resulting in the most favourable hemodynamics of the heart. In this embodiment, a lead 11 is placed in the right ventricle and the sensing means 14 includes a right ventricular pressure sensor arranged to sense the pressure in the right ventricle. The measuring means 16 comprises integrating means arranged to integrate the signal corresponding to the sensed pressure between a start time and a stop time to produce a integration result corresponding to the cardiac output. Moreover, the measuring means 16 comprises filtering means arranged to filter the pressure signal during a systolic phase to identify the opening of a valve at the right ventricle as the start time for the integration and to identify the closing of this valve as the stop time. The details of the design and function of the right ventricular pressure sensor, the integrating means and the filtering means are described in U.S. Pat. No. 6,314,323. During the evaluation procedure, the measuring means 16 records the cardiac output at each lead position, the results are stored in the storing means 17 and transferred to the processing means 18. The information regarding the positions of the lead is input into the evaluation device 15, for example, by the physician or by attendant personnel via the operator unit 31 as discussed above. As also mentioned above, the results can be presented for the physician during the implantation procedure on the screen 33. Then, when cardiac output has been determined for all lead positions, the processing means 18 is arranged to determine the lead position resulting in the highest cardiac output as the lead position resulting in the most favourable hemodynamics of the heart.

According to another embodiment of the present invention, the sensing means 14 mounted within the lead 11 includes one or more accelerometer-based cardiac wall motion sensors. The cardiac wall motion sensors transducer accelerations of cardiac tissue to which the lead 11 is placed in close proximity to or attached, so as to provide one or more signals indicative of cardiac mechanical activity. The cardiac wall motion sensor signals are recorded by the measuring means 16 and are transferred to the processing means 18 in which the signals are processed to derive cardiac wall tissues signals including cardiac wall velocity signals and cardiac wall displacement signals. The derived signals are further processed to derive physiologic parameters indicative of cardiac performance, including stroke volume, contractility, pre-ejection period, and ejection time. Preferably, one or more accelerometer-based cardiac wall motion sensors are arranged at the coronary sinus electrode, which is placed in a coronary vein. Thereby, cardiac tissues motion signals indicative of, for example, left ventricular ejection time and pre-ejection period can be collected and recorded for each lead position, and the processing means 18 can determine the PEP/LVET quotient for the corresponding lead positions. There can be one accelerometer that is moved to different positions within the heart, evaluation is performed at each position and each position is manually indicated by the operator instead of using a multiaccelerometer catheter. Thereafter, the processing means 18 is capable of determining the lead position resulting in the lowest value of the PEP/LVET quotient as the lead position resulting in the most favourable hemodynamics of the heart. The details of the function and design of the sensor and the processing steps to derive the physiologic parameters are described in U.S. Pat. No. 5,628,777 and 5,549,650. The information regarding the positions of the lead is input into the evaluation device 15 in accordance with the above discussion. As also mentioned above, the results can be presented for the physician during the implantation procedure on the screen 33. In an alternative embodiment, one or more accelerometer-based cardiac wall motion sensors is arranged within a lead placed in the right ventricle in addition to the one or more accelerometer-based cardiac wall motion sensors arranged within lead placed in the coronary vein. Thereby, the contraction of the left ventricle and the right ventricle, respectively, or, in other words, the timing difference or delay between the contraction of the left ventricle and the right ventricle and/or the proportion between the left ventricular pressure and the right ventricular pressure can be measured at each lead position and the processing means can determine the lead position resulting in the best co-ordination, i.e. the shortest delay between the contraction of the left ventricle and right ventricle, and/or the best proportionality between the left ventricular pressure and the right ventricular pressure as the lead position resulting in the most favourable hemodynamics of the heart. As mentioned above, the information regarding the positions of the lead are input into the evaluation device 15, for example, by the physician or by attendant personnel via the operator unit 31 and the results can be presented for the physician during the implantation procedure on the screen 33.

Preferably, the AV interval between stimulation of the atrium and the ventricle and/or the W interval between stimulation of the right and left ventricles are optimised before the measurement related to the determination of the performance of the actual lead position is performed in the above mentioned different embodiments of the present invention employing different approaches to derive physiologic parameters reflecting hemodynamic performance for different lead positions in order to determine the optimal lead site.

It should be noted that in the different embodiments of the present invention described above, electrical signals, for example, IEGM signals can be used in combination with the signals indicative of the cardiac mechanical activity obtained by means of acoustic sensors, accelerometer-based sensors, or pressure sensors to produce a hemodynamical measure of the lead positions so as to evaluate the different positions in order to identify the optimal position of the lead or the leads.

Although an exemplary embodiment of the present invention has been shown and described, it will be apparent to those having ordinary skill in the art that a number of changes, modifications, or alterations to the inventions as described herein may be made. Thus, it is to be understood that the above description of the invention and the accompanying drawings is to be regarded as a non-limiting example thereof and that the scope of protection is defined by the appended patent claims. As an example, many of the functions described above may be obtained and carried out by suitable software comprise in a micro-chip, an ASIC, or the like data carrier.

## Claims

1. Device for evaluating positions of a medical lead (11) connectable to said device during an implantation procedure,
said medical lead (11) comprising at least one electrode (13) for stimulating and sensing,
said medical lead (11) comprising at least one mechanical sensor (14), said sensor (14) being arranged to provide signals related to heart pumping activity,
said device (15) comprising:
measuring means (16) arranged to record sensor signals from said lead at different sensor positions;
storage means (17) arranged to store said sensor signal; and
processing means (18) arranged to calculate, for each lead position, a measure of heart activity based on said sensor signal and determine, based on said measures, the lead position resulting in the most favourable hemodynamics of the heart, **characterized in that**
said measuring means (16) is arranged to record IEGM signals from said electrode (13) at each lead position,
said storage means (17) is arranged to store said IEGM signals,
said processing means (18) is arranged to calculate, for each lead position, said measure based on said sensor signal and said IEGM signal,
said storage means (17) is arranged to store said measures together with position data for the corresponding lead position, and
said device comprises a display (33) arranged to present said measures and said lead position resulting in the most favourable hemodynamics of said heart.

2. Device according to claim 1, said recorded signal being a pressure signal from a pressure sensor in said lead (11).

3. Device according to claim 2, one sensor position being in the right ventricle.

4. Device according to claim 3, another sensor position being in the left ventricle or in the coronary sinus.

5. Device according to any one of the preceding claims, wherein said measuring means (16) is arranged to record hemodynamic pressure signals at each lead position.

6. Device according to claim 5, said hemodynamic pressure signals being ventricular pressure signals.

7. Device according to claim 1, said sensors (14) being accelerometer-based sensors.

8. Device according to claim 1, wherein said measuring means (16) is arranged to record signals indicating cardiac mechanical activity at each lead position.

9. Device according to claim 8, wherein said processing means (18) is arranged to process said cardiac mechanical activity signals to derive physical parameters indicative of the cardiac performance.

10. Device according to claim 1, 8 or 9, wherein the processing means (18) is arranged to determine the lead position resulting in the highest ventricular contractility as the lead position resulting in the most favourable hemodynamics of the heart.

11. Device according to claim 10, wherein the highest ventricular contractility is determined as the peak left ventricular pressure change during systole or as the maximum LV + dp/dt.

12. Device according to claim 2, 5, 6, 7, 8 or 9, wherein said processing means (18) is arranged to determine the lead position resulting in the maximum left ventricle pressure as the lead position resulting in the most favourable hemodynamics of the heart.

13. Device according to claim 3, 5, 6, 7, 8, or 9, wherein the lead position resulting in the highest cardiac output is determined to be the lead position resulting in the most favourable hemodynamics of the heart.

14. Device according to claim 4, 5, 6, 7, 8, or 9, wherein the processing means (18) is arranged to determine the lead position resulting in the shortest delay between the contraction of the left ventricle and right ventricle, and/ or a proportionality between the left ventricular pressure and the right ventricular pressure as defined by the user as the lead position resulting in the most favourable hemodynamics of the heart.

15. Device according to claim 1, 5, 6, 7, 8, or 9, wherein said processing means (18) is arranged to determine the lead position resulting in the shortest pre-ejection time period (PEP) to be the lead position resulting in the most favourable hemodynamics of the heart.

16. Device according to claim 15, wherein the PEP is determined as the period from the onset of a QRS or an emitted ventricular stimulation until the opening of the aortic valve.

17. Device according to claim 1, 5, 6, 7, 8, or 9, wherein said processing means (18) is arranged to determine the lead position resulting in the lowest value of the quotient between PEP and left ventricular ejection time (LVET) to be the lead position resulting in the most favourable hemodynamics of the heart.

18. Device according to claim 17, wherein the PEP is determined as the period from the onset of a QRS or an emitted ventricular stimulation until the opening of the aortic valve and the LVET is determined as the period of time required for the systolic ejection of the left ventricle.

19. Device according to any one of claim 17 or 18, said medical lead 11 further comprising an acoustic sensor (14) arranged on said lead (11) being arranged to sense acoustic signals, whereby the opening and/ or closing of the aortic valve can be determined from signals received from said acoustic sensor (14) sensitive to acoustic signals.

20. Device according to any one of preceding claims, connectable to at least one medical lead for placement in the right ventricle and one medical lead placed for stimulating the left ventricle.

21. Device according to claim 20, wherein the VV interval between stimulation of the right and left ventricles is optimized before the measurement related to the determination of the performance of the actual lead position is performed.

22. Device according to any one of preceding claims, connectable to at least one medical lead for placement in a ventricle, said device further being connectable to at least one lead for placement in an atrium.

23. Device according to claim 22, wherein the AV interval between stimulation of the atrium and the ventricle is optimised before the measurement related to the determination of the performance of the actual lead position is performed.

24. Device according to any one of preceding claims, connectable to at least one medical lead for placement in the right ventricle and one medical lead placed for stimulating the left ventricle and at least one lead placed for stimulating an atrium.

25. Device according to claim 24 wherein the AV interval between stimulation of the atrium and the ventricle and the VV interval between stimulation of the right and left ventricles are optimised before the measurement related to the determination of the performance of the actual lead position is performed.

## Patentansprüche

1. Vorrichtung zum Beurteilen von Positionen eines medizinischen Drahts (11), der mit der Vorrichtung während einer Implantationsprozedur verbindbar ist,
wobei der medizinische Draht (11) zumindest eine Elektrode (13) zum Stimulieren und Erfassen umfasst,
wobei der medizinische Draht (11) zumindest einen mechanischen Sensor (14) umfasst, wobei der Sensor (14) ausgelegt ist, Signale bereitzustellen, die sich auf eine Herzpumpaktivität beziehen,
wobei die Vorrichtung (15) umfasst:
eine Messeinrichtung (16), die ausgelegt ist, Sensorsignale von dem Draht an unterschiedlichen Sensorpositionen aufzuzeichnen;
eine Speichereinrichtung (17), die ausgelegt ist, das Sensorsignal zu speichern; und
eine Verarbeitungseinrichtung (18), die ausgelegt ist, für jede Drahtposition ein Maß einer Herzaktivität auf Grundlage des Sensorsignals zu berechnen und auf der Grundlage der Messungen die Drahtposition zu bestimmen, die zu der günstigsten Hämodynamik des Herzens führt,
**dadurch gekennzeichnet, dass**
die Messeinrichtung (16) ausgelegt ist, IEGM-Signale von der Elektrode (13) an jeder Drahtposition aufzuzeichnen,
die Speichereinrichtung (17) ausgelegt ist, die IEGM-Signale zu speichern,
die Verarbeitungseinrichtung (18) ausgelegt ist, für jede Drahtposition das Maß auf Grundlage des Sensorsignals und des IEGM-Signals zu berechnen,
die Speichereinrichtung (17) ausgelegt ist, die Maße zusammen mit Positionsdaten für die entsprechende Drahtposition zu speichern, und
die Vorrichtung eine Anzeigeeinheit (33) umfasst, die ausgelegt ist, die Maße und die Drahtposition aufzuzeigen, die zu der günstigsten Hämodynamik des Herzens führen.

2. Vorrichtung nach Anspruch 1, wobei das aufgezeichnete Signal ein Drucksignal von einem Drucksensor in dem Draht (11) ist.

3. Vorrichtung nach Anspruch 2, wobei eine Sensorposition in dem rechten Ventrikel ist.

4. Vorrichtung nach Anspruch 3, wobei eine weitere Sensorposition in dem linken Ventrikel oder in dem Koronarsinus ist.

5. Vorrichtung nach einem der voranstehenden Ansprüche, wobei die Messeinrichtung (16) ausgelegt ist, hämodynamische Drucksignale an jeder Drahtposition aufzuzeichnen.

6. Vorrichtung nach Anspruch 5, wobei die hämodynamischen Drucksignale ventrikuläre Drucksignale sind.

7. Vorrichtung nach Anspruch 1, wobei die Sensoren (14) beschleunigungsaufnehmer-basierte Sensoren sind.

8. Vorrichtung nach Anspruch 1, wobei die Messeinrichtung (16) ausgelegt ist, Signale aufzuzeichnen, die eine mechanische Herzaktivität an jeder Drahtposition anzeigen.

9. Vorrichtung nach Anspruch 8, wobei die Verarbeitungseinrichtung (18) ausgelegt ist, die mechanischen Herzaktivitätssignale zu verarbeiten, um physikalische Parameter herzuleiten, die anzeigend für eine Herzperformanz sind.

10. Vorrichtung nach Anspruch 1, 8 oder 9, wobei die Verarbeitungseinrichtung (18) ausgelegt ist, die Drahtposition, die zu der höchsten ventrikulären Kontraktionsfähigkeit führt, als die Drahtposition zu bestimmen, die zu der günstigsten Hämodynamik des Herzens führt.

11. Vorrichtung nach Anspruch 10, wobei die höchste ventrikuläre Kontraktionsfähigkeit als die linke ventrikuläre Spitzendruckänderung während einer Systole oder als das Maximum LV + dp/dt bestimmt ist.

12. Vorrichtung nach Anspruch 2, 5, 6, 7, 8 oder 9, wobei die Verarbeitungseinrichtung (18) ausgelegt ist, die Drahtposition, die zu dem maximalen linken ventrikulären Druck führt, als die Drahtposition zu bestimmen, die zu der günstigsten Hämodynamik des Herzens führt.

13. Vorrichtung nach Anspruch 3, 5, 6, 7, 8 oder 9, wobei die Drahtposition, die zu der höchsten Herzleistung führt, als die Drahtposition bestimmt wird, die zu der günstigsten Hämodynamik des Herzens führt.

14. Vorrichtung nach Anspruch 4, 5, 6, 7, 8 oder 9, wobei die Verarbeitungseinrichtung (18) ausgelegt ist, die Drahtposition, die zu der kürzesten Verzögerung zwischen der Kontraktion des linken Ventrikels und des rechten Ventrikels, und/oder einer Proportionalität, je nach Anwenderdefinition, zwischen dem linken ventrikulären Druck und dem rechten ventrikulären Druck führt, als die Drahtposition zu bestimmen, die zu der günstigsten Hämodynamik des Herzens führt.

15. Vorrichtung nach Anspruch 1, 5, 6, 7, 8 oder 9, wobei die Verarbeitungseinrichtung (18) ausgelegt ist, die Drahtposition, die zu der kürzesten Vor-Ausstoß-Zeitperiode (Pre-Ejection Time Period, PEP) führt, als die Drahtposition zu bestimmen, die zu der günstigsten Hämodynamik des Herzens führt.

16. Vorrichtung nach Anspruch 15, wobei die PEP als die Periode von dem Beginn eines QRS oder einer emittierten ventrikulären Stimulation bis zum Öffnen der Aortenklappe bestimmt ist.

17. Vorrichtung nach Anspruch 1, 5, 6, 7, 8 oder 9, wobei die Verarbeitungseinrichtung (18) ausgelegt ist, die Drahtposition, die zu dem niedrigsten Wert des Quotienten zwischen der PEP und der linken ventrikulären Ausstoßzeit (Left Ventricular Ejection Time, LVET) führt, als die Drahtposition zu bestimmen, die zu der günstigsten Hämodynamik des Herzens führt.

18. Vorrichtung nach Anspruch 17, wobei die PEP als die Periode von dem Beginn eines QRS oder einer emittierten ventrikulären Stimulation bis zu dem Öffnen der Aortenklappe bestimmt ist, und die LVET als die Zeitperiode bestimmt ist, die für den systolischen Ausstoß des linken Ventrikels erforderlich ist.

19. Vorrichtung nach einem der Ansprüche 17 oder 18, wobei der medizinische Draht (11) ferner einen an dem Draht (11) angeordneten akustischen Sensor (14) umfasst, der ausgelegt ist, akustische Signale zu erfassen, wodurch das Öffnen und/oder Schließen der Aortenklappe aus Signalen bestimmt werden kann, die von dem akustischen Sensor (14) empfangen werden, der empfindlich auf akustische Signale ist.

20. Vorrichtung nach einem der voranstehenden Ansprüche, verbindbar mit zumindest einem medizinischen Draht zur Platzierung in dem rechten Ventrikel und einem medizinischen Draht, der zum Stimulieren des linken Ventrikels platziert ist.

21. Vorrichtung nach Anspruch 20, wobei das VV-Intervall zwischen einer Stimulation der rechten und linken Ventrikel optimiert wird, bevor die Messung, die sich auf die Bestimmung der Performanz der tatsächlichen Drahtposition bezieht, durchgeführt wird.

22. Vorrichtung nach einem der voranstehenden Ansprüche, verbindbar mit zumindest einem medizinischen Draht zur Platzierung in einem Ventrikel, wobei die Vorrichtung ferner mit zumindest einem Draht zur Platzierung in einem Vorhof verbindbar ist.

23. Vorrichtung nach Anspruch 22, wobei das AV-Intervall zwischen einer Stimulation des Vorhofs und des Ventrikels optimiert wird, bevor die Messung, die sich auf die Bestimmung der Performanz der tatsächlichen Drahtposition bezieht, durchgeführt wird.

24. Vorrichtung nach einem der voranstehenden Ansprüche, verbindbar mit zumindest einem medizinischen Draht zur Platzierung in dem rechten Ventrikel und einem medizinischen Draht, der zum Stimulieren des linken Ventrikels platziert ist, und zumindest einem Draht, der zum Stimulieren eines Vorhofs platziert ist.

25. Vorrichtung nach Anspruch 24, wobei das AV-Intervall zwischen einer Stimulation des Vorhofs und des Ventrikels und das VV-Intervall zwischen einer Stimulation der rechten und linken Ventrikel optimiert werden, bevor die Messung, die sich auf die Bestimmung der Performanz der tatsächlichen Drahtposition bezieht, durchgeführt wird.

## Revendications

1. Dispositif pour évaluer les positions d'une dérivation médicale (11) connectable audit dispositif pendant une procédure d'implantation,
ladite dérivation médicale (11) comprenant au moins une électrode (13) de stimulation et de détection,
ladite dérivation médicale (11) comprenant au moins un capteur mécanique (14), ledit capteur (14) étant configuré pour fournir des signaux relatifs à l'activité du pompage cardiaque,
ledit dispositif (15) comprenant :
un moyen de mesure (16) configuré pour enregistrer des signaux de capteur provenant de ladite dérivation dans différentes positions de capteur ;
un moyen de stockage (17) configuré pour stocker ledit signal de capteur ; et
un moyen de traitement (18) configuré pour calculer, pour chaque position de dérivation, une mesure de l'activité cardiaque sur la base dudit signal de capteur, et pour déterminer, sur la base desdites mesures, la position de dérivation donnant lieu à l'hémodynamique la plus favorable du coeur, **caractérisé en ce que**
ledit moyen de mesure (16) est configuré pour enregistrer des signaux d'ECG endocavitaire provenant de ladite électrode (13) dans chaque position de dérivation,
ledit moyen de stockage (17) est configuré pour stocker lesdits signaux d'ECG endocavitaire,
ledit moyen de traitement (18) est configuré pour calculer, pour chaque position de dérivation, ladite mesure sur la base dudit signal de capteur et dudit signal d'ECG endocavitaire,
ledit moyen de stockage (17) est configuré pour stocker lesdites mesures conjointement avec les données de position de la position de dérivation correspondante, et
ledit dispositif comprend un écran (33) configuré pour présenter lesdites mesures et ladite position de dérivation donnant lieu à l'hémodynamique la plus favorable dudit coeur.

2. Dispositif selon la revendication 1, dans lequel ledit signal enregistré est un signal de pression provenant d'un capteur de pression dans ladite dérivation (11).

3. Dispositif selon la revendication 2, dans lequel une position de capteur se trouve dans le ventricule droit.

4. Dispositif selon la revendication 3, dans lequel une autre position de capteur se trouve dans le ventricule gauche ou dans le sinus coronaire.

5. Dispositif selon l'une quelconque des revendications précédentes, dans lequel ledit moyen de mesure (16) est configuré pour enregistrer les signaux de pression hémodynamique dans chaque position de dérivation.

6. Dispositif selon la revendication 5, dans lequel lesdits signaux de pression hémodynamique sont des signaux de pression ventriculaire.

7. Dispositif selon la revendication 1, dans lequel lesdits capteurs (14) sont des capteurs de type accéléromètre.

8. Dispositif selon la revendication 1, dans lequel ledit moyen de mesure (16) est configuré pour enregistrer des signaux indiquant l'activité mécanique cardiaque dans chaque position de dérivation.

9. Dispositif selon la revendication 8, dans lequel ledit moyen de traitement (18) est configuré pour traiter lesdits signaux d'activité mécanique cardiaque afin de déterminer des paramètres physiques révélateurs de la performance cardiaque.

10. Dispositif selon la revendication 1, 8 ou 9, dans lequel le moyen de traitement (18) est configuré pour déterminer la position de dérivation donnant lieu à la plus forte contractilité ventriculaire comme étant la position de dérivation donnant lieu à l'hémodynamique la plus favorable du coeur.

11. Dispositif selon la revendication 10, dans lequel la plus forte contractilité ventriculaire est déterminée comme étant le pic de changement de pression ventriculaire gauche pendant la systole ou comme étant la valeur LV + dp/dt maximale.

12. Dispositif selon la revendication 2, 5, 6, 7, 8 ou 9, dans lequel ledit moyen de traitement (18) est configuré pour déterminer la position de dérivation donnant lieu à la pression ventriculaire gauche maximale comme étant la position de dérivation donnant lieu à l'hémodynamique la plus favorable du coeur.

13. Dispositif selon la revendication 3, 5, 6, 7, 8 ou 9, dans lequel la position de dérivation donnant lieu au débit cardiaque maximal est déterminée comme étant la position de dérivation donnant lieu à l'hémodynamique la plus favorable du coeur.

14. Dispositif selon la revendication 4, 5, 6, 7, 8 ou 9, dans lequel le moyen de traitement (18) est configuré pour déterminer la position de dérivation donnant lieu au délai le plus court entre la contraction du ventricule gauche et du ventricule droit et/ou à une proportionnalité entre la pression ventriculaire gauche et la pression ventriculaire droite, telle que définie par l'utilisateur comme étant la position de dérivation donnant lieu à l'hémodynamique la plus favorable du coeur.

15. Dispositif selon la revendication 1, 5, 6, 7, 8 ou 9, dans lequel ledit moyen de traitement (18) est configuré pour déterminer la position de dérivation donnant lieu à la phase de pré-éjection (PEP) la plus courte comme étant la position de dérivation donnant lieu à l'hémodynamique la plus favorable du coeur.

16. Dispositif selon la revendication 15, dans lequel la PEP est déterminée comme étant la période allant du début d'un QRS ou d'une stimulation ventriculaire émise à l'ouverture de la valve aortique.

17. Dispositif selon la revendication 1, 5, 6, 7, 8 ou 9, dans lequel ledit moyen de traitement (18) est configuré pour déterminer la position de dérivation donnant lieu à la valeur la plus faible du quotient entre la PEP et le temps d'éjection ventriculaire gauche (TEVG) comme étant la position de dérivation donnant lieu à l'hémodynamique la plus favorable du coeur.

18. Dispositif selon la revendication 17, dans lequel la PEP est déterminée comme étant la période allant du début d'un QRS ou d'une stimulation ventriculaire émise à l'ouverture de la valve aortique, et le TEVG est déterminé comme étant la période de temps requise pour l'éjection systolique du ventricule gauche.

19. Dispositif selon l'une quelconque de la revendication 17 ou 18, dans lequel ladite dérivation médicale (11) comprend en outre un capteur acoustique (14) disposé sur ladite dérivation (11) et configuré pour détecter des signaux acoustiques, et dans lequel l'ouverture et/ou la fermeture de la valve aortique peuvent être déterminées à partir des signaux reçus dudit capteur acoustique (14) sensible aux signaux acoustiques.

20. Dispositif selon l'une quelconque des revendications précédentes, connectable au moins à une dérivation médicale pour une mise en place dans le ventricule droit et à une dérivation médicale mise en place pour stimuler le ventricule gauche.

21. Dispositif selon la revendication 20, dans lequel l'intervalle VV entre la stimulation des ventricules droit et gauche est optimisé avant que la mesure relative à la détermination de la performance de la position de dérivation réelle ne soit réalisée.

22. Dispositif selon l'une quelconque des revendications précédentes, connectable à au moins une dérivation médicale pour une mise en place dans un ventricule, ledit dispositif étant en outre connectable à au moins une dérivation pour une mise en place dans un atrium.

23. Dispositif selon la revendication 22, dans lequel l'intervalle AV entre la stimulation de l'atrium et du ventricule est optimisé avant que la mesure relative à la détermination de la performance de la position de dérivation réelle ne soit réalisée.

24. Dispositif selon l'une quelconque des revendications précédentes, connectable au moins à une dérivation médicale pour une mise en place dans le ventricule droit et à une dérivation médicale mise en place pour stimuler le ventricule gauche, et à au moins une dérivation mise en place pour stimuler un atrium.

25. Dispositif selon la revendication 24, dans lequel l'intervalle AV entre la stimulation de l'atrium et du ventricule et l'intervalle VV entre la stimulation des ventricules droit et gauche sont optimisés avant que la mesure relative à la détermination de la performance de la position de dérivation réelle ne soit réalisée.
